# EUROPEAN PATENT SPECIFICATION

(11) **EP 1 352 085 B1**
(45) Date of publication and mention of the grant of the patent: **15.04.2009**
(21) Application number: 01270623.0
(22) Date of filing: 11.12.2001
(51) Int. Cl.: C12Q 1/68, G01N 33/53, G01N 33/574

(54) **P2Y PURINERGIC RECEPTOR EXPRESSION FOR IDENTIFYING PRENEOPLASTIC AND NEOPLASTIC STATES**
EXPRESSION VON P2Y-PURINERGEM REZEPTOR ZUR IDENTIFIZIERUNG PRÄNEOPLASTISCHER UND NEOPLASTISCHER ZUSTÄNDE
EXPRESSION DU RECEPTEUR P2Y PURINERGIQUE POUR IDENTIFIER DES ETATS PRENEOPLASTIQUES ET NEOPLASTIQUES

(30) Priority: 11.12.2000 AU PR201500
(43) Date of publication of application: 15.10.2003
(73) Proprietor: Biosceptre Pty Ltd, Sydney, NSW 2000 (AU)
(72) Inventor: BARDEN, Julian, Marsfield, NSW 2122 (AU); SLATER, Michael, Belmore, NSW 2192 (AU)
(74) Representative: MacLean, Martin Robert
(86) International application number: PCT/AU2001/001614
(87) International publication number: WO 2002/048395

(56) References cited:
- WO-A1-01/06259
- WO-A2-01/30964
- AYYANATHAN KASIRAJAN ET AL: "Cloning and chromosomal localization of the human P2Y1 purinoceptor" BIOCHEMICAL AND BIOPHYSICAL RESEARCH COMMUNICATIONS, ACADEMIC PRESS INC. ORLANDO, FL, US, vol. 218, no. 3, 1996, pages 783-788, XP002195807 ISSN: 0006-291X
- KING B F ET AL: "Metabotropic receptors for ATP and UTP: exploring the correspondence between native and recombinant nucleotide receptors" TRENDS IN PHARMACOLOGICAL SCIENCES, ELSEVIER TRENDS JOURNAL, CAMBRIDGE, GB, vol. 19, no. 12, December 1998 (1998-12), pages 506-514, XP004149716 ISSN: 0165-6147
- JANSSENS R., BOEYNAEMS J.M.: 'Effects of extracellular nucleotides and nucleosides on prostate carcinoma cells' BR. J. PHARMACOL. vol. 132, no. 2, January 2001, pages 536 - 546, XP002973033
- WAGSTAFF S.C. ET AL: 'Extracellular ATP activates multiple signalling pathways and potentiates growth factor-induced c-fos gene expression in MCF-7 breast cancer cells' CARCINOGENESIS vol. 21, no. 12, December 2000, pages 2175 - 2181, XP002973032
- SCHULTZE-MOSGAU A. ET AL: 'Characterization of calcium-mobiliziung, purinergic P2Y(2) receptors in human ovarian cancer cells' MOL. HUMAN REPRODUCT. vol. 6, no. 5, May 2000, pages 435 - 442, XP002961943
- KATZUR A.C. ET AL: 'Expression and responsiveness of P2Y2 receptors in human endometrial cancer cell lines' J. CLIN. ENDOCRINOL. METAB. vol. 84, no. 11, November 1999, pages 4085 - 4091, XP002973035
- HOEPFNER M. ET AL: 'Expression of functional P2-purinergic receptors in primary cultures of human colorectal carcinoma cells' BIOCHEM. BIOPHYS. RES. COMM. vol. 251, no. 3, 1998, pages 811 - 817, XP001066524
- SAUER H. ET AL: 'Calcium-dependence of hydrogen peroxide-induced c-fos expression and growth stimulation of multicellular prostate tumor spheroids' FEBS LETTERS vol. 419, no. 2-3, 1997, pages 201 - 205, XP004261619

## Description

### TECHNICAL FIELD

The present invention relates to in vitro methods for identifying pre-neoplastic and neoplastic states in mammals and in particular to an in vitro method for identifying pre-neoplasia and neoplasia in cells and tissues based on differential expression of P2Y purinergic receptors.

### BACKGROUND

When diagnosing cancer, cellular features in biopsy samples are taken into account such as the degree of variability of cancer cell size and shape, the proportion of actively dividing cells and invasion into neighbouring structures. Commonly used histological stains are haematoxylin (primary stain) and eosin (counterstain) which differentially label subcellular elements. Other diagnostic methods employ antibodies to particular diagnostic molecules within (via intracellular epitopes) or on the surface of cells or tissues (via extracellular epitopes) which can be made visible for microscopic analysis eg, carcino-embryonic antigen (CEA). Some specific examples are discussed below.

### Prostate Cancer

The incidence of prostate cancer in the Western world is increasing at an alarming rate, having more than doubled in the past five years. It has the highest incidence of any neoplasm, is second only to lung cancer as the most common cause of cancer death in men worldwide, and is the leading cause of cancer death in Australia [1]. Benign prostatic hyperplasia (BPH) is common in men over 50 and is a possible precursor of prostatic intraepithelial neoplasia (PIN), itself a precursor to prostate cancer. Postmortem studies indicate that 70% of men have malignant cells in their prostate by the time they reach 80 [2].

Despite the gravity of this condition, diagnostic methods are few and imprecise. Current methods for assessing prognosis, such as digital rectal examination (DRE), ultrasound, prostatic acid phosphatase levels, androgen ablation, prostate specific antigen (PSA) density, PSA velocity, PSA age-specific reference ranges and Gleason histopathological grading, can fail to provide reliable predictive information regarding the clinical outcome of prostate cancer [3]. For instance, studies have shown that DRE results in a 36.9% false negative rate [4]. PSA is a 33-kDa serine protease that is associated with a number of tissues besides prostate [5], is up-regulated by androgens, glucocorticoids and progestins and is thought to be involved in the regulation of growth factors. Unfortunately, serum PSA levels have an incidence of 23% false negative and 36.7% false positive diagnoses [5]. It has even been suggested that more than half of new screen-detected cases are in fact false positives [6]. Attempts to improve screening methods by the introduction of additional tests such as PSA density, velocity, and age-specific reference ranges has been equivocal. One study has shown that applying an age-specific PSA reference range that increases the upper limit of normal PSA to 4.5 ng/mL results in the failure to detect a substantial number of clinically significant cancers [7]. Given this uncertainty, prostate biopsy is often performed to confirm malignancy but this test also has a highly unsatisfactory 23% incidence of false-negative diagnosis [8], caused by the failure to directly sample the site of neoplastic change.

Treatment selection is largely dependent on clinical staging based on microscopic analysis of tissue sections [9]. This technique depends on judgment and considerable experience in relating histological appearance to clinical outcome. Unfortunately, prostate cancer tissue is notoriously heterogeneous and a vital diagnostic feature may easily be missed in the section being examined. To further complicate the situation, there have been no randomised and controlled trials to examine the outcomes of surgery and radiotherapy [2]. Treatment choices include radical prostatectomy, radiation therapy, androgen deprivation and "watchful waiting". A definitive answer to the question of "watchful waiting" versus radical intervention awaits the conclusion of the prostate cancer intervention-versus-observation trial [10]. The consequences to the patient of these decisions are serious. Radical prostatectomy for instance, often results in incontinence, impotence, bladder neck stricture, pain and depression [11].

In the case of other cancers such as breast or skin, samples can be misdiagnosed for several reasons. In the case of breast tumours, the borders of a localised tumour may not be well defined and similarly in the case of skin cancers, the borders between histologically obvious cancer (as determined with H&E stain) and normal tissue are variable. Either more or less normal tissue is excised around these tumours than is desirable.

It is an object of the present invention to provide an in vitro method of identifying pre-neoplastic and/or neoplastic cells which will overcome or substantially ameliorate at least some of the deficiencies of the prior art or will provide a useful alternative.

WO/2000/050458 describes the cloning of a P2Y-like 7TM receptor named AXOR17, and AXOR17 polypeptides and polynucleotides. Also described are methods for producing AXOR17 polypeptides and polynucleotides using recombinant techniques, methods for using such polypeptides and polynucleotides 113 therapy, and diagnostic assays.

The present invention provides an in vitro method of diagnosing or staging a preneoplastic and/or neoplastic state in a mammal, or determining the aetiology of carcinogenesis in a mammal, comprising detection of the P2Y purinergic receptor expression profile of a cell and/or tissue isolated from said mammal and comparison of the profile with a predetermined expression profile of a normal cell and/or tissue.

The present invention also provides a P2Y purinergic receptor-specific antibody, for use in differentiating between a pre-neoplastic or neoplastic cell and/or tissue and a normal cell and/or tissue.

The present invention further provides a suite of P2Y purinergic receptor-specific antibodies, for use in differentiating between a pre-neoplastic or neoplastic cell and/or tissue and a normal cell and/or tissue, comprising antibodies as described above.

### SUMMARY OF THE INVENTION

P2Y purinergic receptors are G-protein coupled tissue receptors which bind adenosine triphosphate (ATP) and, in the case of some P2Y receptor subtypes, uridine triphosphate (UTP). It has surprisingly been found that the expression pattern of P2Y receptors can be used to diagnose pre-neoplasia and neoplasia in mammals.

Pre-cancer and cancer stages in mammals are accompanied by marked differences in growth, extracellular matrix, metabolic and innervation factors as well as increases in subepithelial ionic calcium and microtubules. Using the novel antibodies of the present invention, the P2Y receptors can be readily visualised with immunocytochemical methods and it has been shown that they present in a variety of expression patterns such as cell surface, tubular and punctate labelling. These expression patterns can be used to identify the different stages in cancer development, commencing with entirely normal tissue and progressing through preneoplasia where P2Y receptor expression in the prostate as an example first appears in the nuclei within individual affected acini. In more advanced preneoplasia, but where there are still no morphological signs of disease, the receptors leave the nuclei and spread into the cytoplasm and lateral and basal membranes within the acini prior to deposition on the apical membrane of the epithelial cells, an event occurring in synchrony with the morphological changes accompanying early cancer. Since these stages are more global in nature than the morphologically defined areas of obvious tumour, the diagnostic is not confined to direct sampling of the area of immediate tumour but can detect the tumour presence at some distance.

The application therefore describes a new tool with which to diagnose pre-cancerous conditions (such as hyperplasia), to stage cancer and to investigate the basic physiology and aetiology of carcinogenesis.

According to a first aspect, the invention provides an in vitro method for diagnosing a pre-neoplastic and/or neoplastic state in a mammal, comprising detection of the P2Y purinergic receptor expression profile of a cell and/or tissue from said mammal and comparison of the profile with a predetermined expression profile of a normal cell and/or tissue.

According to a second aspect, the invention provides an in vitro method for staging a pre-neoplastic and/or neoplastic state in a mammal, comprising detection of the P2Y purinergic receptor expression profile of a cell and/or tissue from said mammal and comparison of the profile with a predetermined expression profile of a normal cell and/or tissue.

According to a third aspect, the invention provides an in vitro method of determining the aetiology of carcinogenesis in a mammal, comprising detection of the P2Y purinergic receptor expression profile of a cell and/or tissue from the mammal and comparison of the profile with a predetermined expression profile of a normal cell and/or tissue.

Preferably, the mammal is a human but the method may be applied to any suitable mammal including a horse, a bovine animal, a sheep, a dog, a cat, a rat and a mouse.

Preferably, the cell and/or tissue is from a prostate, breast or skin. However, the skilled addressee will recognise that the method may also be useful for other cells types including other epithelial cells.

When the pre-neoplastic or neoplastic state is prostate pre-cancer or prostate cancer, an increase in the intensity of the P2Y₂ purinergic receptor expression profile in a prostate cell or prostate tissue, compared to the expression profile of a normal prostate cell or tissue, as herein defined, is diagnostic of the presence of prostate pre-cancer or prostate cancer.

When the pre-neoplastic or neoplastic state is breast pre-cancer or breast cancer, a decrease in the intensity of the P2Y₂ purinergic receptor expression profile in a breast cell and/or breast tissue obtained from the mammal compared to the expression profile of a breast cell and/or breast tissue from a normal breast, is diagnostic of the presence of breast pre-cancer or breast cancer.

When the pre-neoplastic or neoplastic state is skin pre-cancer or skin cancer, a decrease in the intensity of the P2Y₂ purinergic receptor expression profile in a skin cell and/or tissue from the mammal compared to the expression profile of a skin cell and/or tissue from normal skin, is diagnostic of the presence of skin pre-cancer or skin cancer.

Preferably, the skin cancer is malignant melanoma, squamous cell carcinoma or basal cell carcinoma. Thus, in accordance with the present invention, it is possible to differentiate skin cancer states, such as those mentioned, from keratoacanthoma and other benign lesions such as actinic keratoses.

Preferably, the P2Y receptor expression profile is the P2Y₂, P2Y₄ and/or P2Y₆ receptor expression profile and more preferably, it is the P2Y₂ receptor expression profile. The skilled addressee, without undue experimentation, can ascertain which P2Y receptors are most suitable for the particular method being practiced. Generally, those P2Y receptors which give the best contrast in terms of the difference in the intensity of expression profile between pre-neoplastic or neoplastic cells or tissues compared to normal cells or tissues are the most desirable.

In one embodiment, the pre-neoplastic state is an early pre-neoplastic state in which the P2Y receptor expression profile is altered in the nucleus of the cell. Alternatively, in another embodiment, the pre-neoplastic state is an advanced pre-neoplastic state in which the P2Y receptor expression profile is a profile in which the P2Y receptors are in the cytoplasm and/or lateral membranes and/or basal membranes.

In another embodiment, the neoplastic state is a state in which the P2Y receptor expression profile is a profile wherein the P2Y receptors are on the apical membrane of an epithelial cell.

It will be clear to the skilled addressee that the P2Y receptor expression profile can be combined with the expression profile of other markers. In particular, the P2Y receptor expression profile can be combined with a P2X receptor expression profile and preferably, with the expression profile of P2X₁ and/or P2X₂.

Preferably, detection of the P2Y receptor expression profile is by immunohistochemical means. However, it will be clear to the skilled addressee that the P2Y receptors may be detected by other means including ELISA, RIA or similar immunological techniques, depending on the source of the cell or tissue sample and the reagents available. Preferably, the P2Y receptors are detected by a colorimetric assay such as DAB (diaminobenzidine) or the LSAB (labelled streptavidin biotin technique) secondary antibody detection kit (Dako).

It will be clear to those skilled in the art that standard Western blotting techniques and standard Northern detection of P2Y purinergic receptor mRNA may also be useful in determining the P2Y receptor expression profile.

According to a fourth aspect, the invention provides a P2Y purinergic receptor-specific antibody, wherein the antibody is capable of differentiating between pre-neoplastic or neoplastic cells and/or tissue and normal cells and/or tissue.

Preferably, the antibody is specific for P2Y₂, P2Y₄ and/or P2Y₆ receptors. Most preferably, the antibody is specific for P2Y₂ receptors.

It will be clear to those skilled in the art the P2Y receptor-specific antibodies may be polyclonal or monoclonal. It will also be clear to those skilled in the art that the P2Y receptor-specific antibody may be combined with other antibodies and, in particular, with one or more P2X receptor-specific antibodies. The P2X receptor-specific antibody may also be monoclonal or polyclonal. In a preferred embodiment, the P2X receptor antibody is a P2X₁ and/or P2X₂ receptor-specific antibody.

According to a fifth aspect, the present invention provides a suite of antibodies comprising antibodies according to the fourth aspect.

According to a sixth aspect, the invention provides a P2Y purinergic receptor-specific antibody according to the fourth aspect or a suite of antibodies according to the fifth aspect when used to differentiate between pre-neoplastic or neoplastic cells and/or tissue and normal cells and/or tissue.

The application describes an isolated mammalian cell or tissue sample complexed with a P2Y purinergic receptor-specific antibody according to the fourth aspect or a suite of antibodies according to the fifth aspect.

The cells and/or tissue for use in the invention may be obtained by biopsy (for example, by fme needle aspirate, especially from breast tissue) but may also be obtained by any other means including from a body fluid or, in the case of prostate cells and/or tissue, from digital rectal examination exudate or from semen.

It will be clear to the person skilled in the art that the methods and P2Y receptor-specific antibodies of the present invention may be used in combination with any other complementary test including, for example, a test for malignancy. The test for malignancy may be an anti-telomerase antibody test using a specific antibody against human telomerase protein 1 (hTP₁) or any other suitable assay or test such as an anti-tenascin test.

The application describes a kit for diagnosing a pre-neoplastic and/or neoplastic state in a mammal comprising means for detecting a P2Y purinergic receptor expression profile.

The application also describes a kit for staging a pre-neoplastic and/or neoplastic state in a mammal comprising means for detecting a P2Y purinergic receptor expression profile.

The application further describes a kit for determining the aetiology of carcinogenesis in a mammal comprising means for detecting a P2Y purinergic receptor expression profile.

Preferably, the means for detecting a P2Y purinergic receptor expression profile is an antibody according to the fourth aspect or a suite of antibodies according to the fifth

Preferably, the kit is used in a method according to any one of the first to third aspects.

In the context of the present invention, the term "neoplastic cell" and "neoplastic tissue" are to be construed in their ordinary sense and include, but are not limited to, a cell or tissue which has the characteristic feature of a neoplastic cell or tissue such as, for example, hyperplasia and/or a hypertrophic growth pattern and the characteristic staining with haematoxylin and eosin stain (ie. H&E) that highlights abnormal cell shape and size and nucleus size

In the context of the present invention, the terms "pre-neoplastic cell" and "pre-neoplastic tissue" are to be construed in their ordinary sense and include, but are not limited to, a cell or tissue that may be hyperplasic or hypertrophic. Such a cell or tissue, however, does not have the characteristic features of a neoplastic cell or tissue such as, for example, the characteristic staining with H&E that highlights abnormal cell shape and size and nucleus size.

In the context of the present invention, the terms "normal cell" and "normal tissue" are to be construed in their ordinary sense and include a cell or tissue in which the P2Y expression profile is not substantially altered from the profile that would be observed in the cells from an individual having neither pre-neoplasia nor neoplasia. In the context of the present invention as it relates to conditions of the prostate, the term "normal cell" also includes, for example, a cell from a prostate having benign prostate hyperplasia.

In the context of the present invention the term "suite of antibodies" comprises polyclonal antibodies which contain several different antibodies specific for the same or different antigens and which are able to specifically differentiate between each of the receptor subtypes. When the antibodies are monoclonal, the term "suite of antibodies" also comprises a panel of antibodies able to specifically differentiate between each of the receptors e.g. P2Y and P2X or the receptor subtypes e.g. P2Y₂ and P2Y₄.

In the context of the present invention, detection of an "expression profile" comprises detection of a pattern or intensity of expression.

Unless the context clearly requires otherwise, throughout the description and the claims, the words 'comprise', 'comprising', and the like are to be construed in an inclusive sense as opposed to an exclusive or exhaustive sense; that is to say, in the sense of "including, but not limited to".

### BRIEF DESCRIPTION OF THE FIGURES

Fig 1 shows prominent epithelial nuclei labelled with P2Y₂ antibody, the first stage of preneoplasia.
Fig 2 shows typical stage 2 staining of preneoplasia where the label now forms a punctate pattern (arrow) with no stain in the nuclei but equally with no morphological evidence yet of cancer.
Fig 3 staining shows that P2Y₂ collects on the apical epithelium (arrow) in tissue showing evidence of morphological change associated with advanced cancer.
Fig. 4 shows a combination of P2Y₂, P2X, and P2X₂ antibody labelling of prostate tissue from a stage 3 case (obvious cancer).
Fig 5 shows non-cancerous breast tissue (fibroadenoma) labelled with a combination of P2Y₂, P2X₁ and P2X₂ antibodies. A small improvement in label intensity is observed with the mixture of labels.
Fig 6 shows a similar serial section of non-cancerous fibroadenoma labelled with P2Y₂ showing closely similar staining.
Fig 7 shows cancerous breast tissue labelled with a combination of P2Y₂, P2X₁ and P2X₂ antibodies. The cancer tissue is devoid of label.
Fig 8 shows breast cancer tissue labelled with P2Y₂ showing closely similar loss of positive staining.
Fig 9 shows an example of a biopsy of a benign lesion in normal skin labelled with P2Y₂ antibody with prominent nuclei (PN) labelled.
Fig 10 shows a biopsy section of malignant melanoma labelled with P2Y₂ antibody in which all the label that had been visible in the normal samples is lost in the melanoma.

Normal label is regained within 2 mm of the melanoma. Combinations of antibodies used above yield identical results.

### DESCRIPTION OF THE INVENTION

The present invention is based on the finding that pre-neoplastic and-neoplastic cells differentially express P2Y purinergic receptors. This differential expression can therefore be used to identify pre-neoplastic or neoplastic cells. Further, because the P2Y purinergic receptors are distributed differently in cells at different stages of pre-neoplasia and neoplasia, the expression profile of the P2Y purinergic receptors can be used to stage pre-neoplastic and neoplastic states. The differential expression patterns of P2Y can also be used to determine the aetiology of cancinogenesis. Further, the application describes a kit for diagnosing a pre-neoplastic and/or neoplastic state comprising means for detecting a P2Y purinergic receptor expression profile; a kit for staging a pre-neoplastic and/or neoplastic state comprising means for detecting a P2Y purinergic receptor expression profile; and a kit for determining the aetiology of carcinogenesis comprising means for detecting a P2Y purinergic receptor expression profile.

Preferred embodiments of the invention will now be described by way of example only.

### Example 1 - Antibody Production

The consensus sequences of the human P2Y₂[12], human P2Y₄[13], and human P2Y₆[14] cloned receptors were examined for suitable epitopes following the approach adopted in Hansen et al. [15]. The non-homologous epitope corresponding to the segment Leu226-Lys242 in P2Y₂ was used, with the comparable sequences in P2Y₄ (Arg226-Arg242) and P2Y₆ (Cys220-Lys236). Each was attached to diphtheria toxoid via maleimidocaproyl-N-hydroxysuccinimide crosslinker through adding an N-terminal Cys to P2Y₂ and P2Y₄ epitopes. All syntheses were carried out using standard t-BOC chemistry on an ABI synthesiser [16]. The peptide-antigen conjugates were suspended in water at 5 mg/mL and aliquots emulsified by mixing with Complete Freund's Adjuvant. Emulsion volumes of 1 mL containing 2 mg of peptide were injected intramuscularly with second, third, fourth and fifth immunisations followed at 2 week intervals using Incomplete Freund's Adjuvant. Final bleeds via venepuncture were obtained at 10-12 weeks, after it was established that adequate antibody titres had been obtained in the rabbits or sheep used for each epitope. The blood was incubated at 37°C for 30 min, and stored at 4°C for 15 h after which the serum was collected following centrifugation and stored at -20°C in small aliquots. Sera were tested with an ELISA assay for antibodies specific for each peptide. The antibody titre, defined as the reciprocal of the serum dilution resulting.in an absorbance of 1.0 above background in the ELISA assay, was in the range 75,000 ± 4,000 compared with 225 ± 25 for the pre-immune samples. Each antibody was found to be specific against the particular subtype.

Affinity purification of each of the antibodies against the specific epitope for that antibody resulted in reduced background but identical labelling trends. A monoclonal antibody raised against the same P2Y₂ epitope in mice yielded the same results.

### Example 2 - Immunohistochemical Procedure

Sections with a thickness of 8 µm were cut from unfixed, frozen tissue using a Reichert Jung 2800 Frigocut cryotome or else were sectioned from paraffin-embedded tissue. Sections were air dried at room temperature for 1 hour, fixed for 12 hours in acetone at -20°C and air dried at room temperature for 1 hour prior to antibody labelling. They were then incubated at room temperature with either a monoclonal mouse, rabbit or sheep anti-P2Y₂ primary antibody. After washing, sections were then incubated for 30 min in the secondary antibody: a 1:30 dilution of HRP-conjugated goat anti-mouse secondary antibody (Dako) for mouse primary, HRP-conjugated anti-rabbit secondary antibody for rabbit primary, or HRP-conjugated goat anti-sheep secondary antibody. Slides were again rinsed and then immersed in 15% diaminobenzidine tetrahydrochloride (DAB - Sigma) for 10 minutes. Sections were rinsed, air dried and mounted in DPX (Merck). Control slides were incubated in diluent buffer during the first incubation and then treated in the same manner as the experimental slides. Negative control slides were treated in the same manner as the experimental slides except that the primary antibody was replaced with non-immune serum.

Individual specificity of all antibodies was demonstrated.

### Example 3 - P2Y receptors in human cancer tissue (prostate)

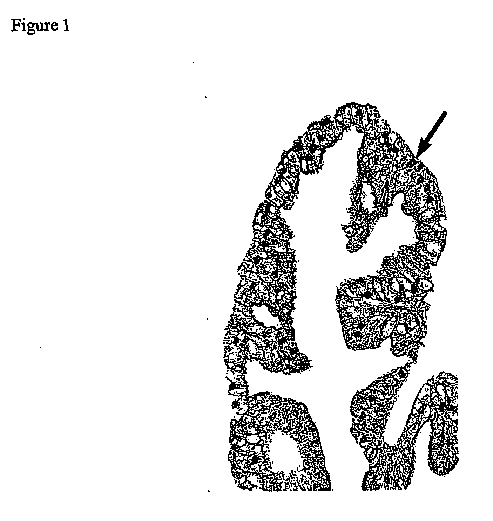

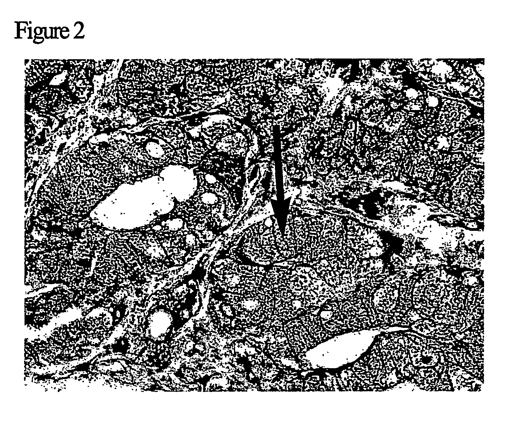

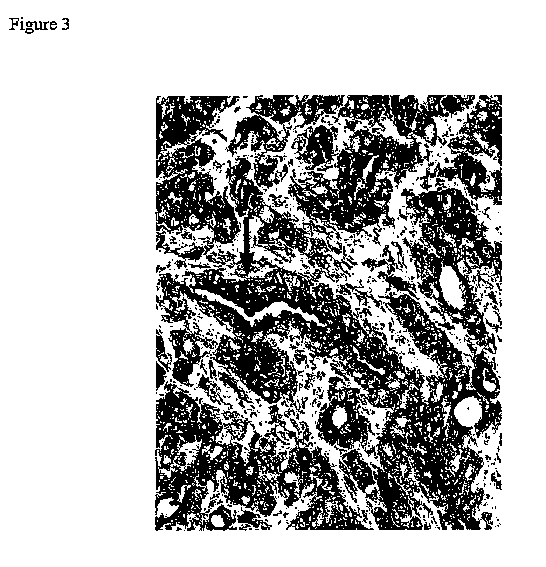

In a study of 40 normal and 40 human prostate cancer cases, P2Y₂ subtypes were markedly increased in human prostate cancer tissue. There was no observable labelling of normal tissue/benign prostatic hyperplasia. The labelling pattern for P2Y₂ in the cancerous tissue was particularly informative in that there was a greater proportion of labelled acinar epithelial cells with each stage of prostate disease, showing a direct correlation between neoplastic transformation and the extent of P2Y₂ acinar labelling commencing with the nuclei (stage 1; Figure 1), progressing to extra-nuclear or cytoplasmic punctate labelling (stage 2; Figure 2) and progressing to deposition on the apical epithelium and lateral epithelium (stage 3; Figure 3) in advanced cases where stage 3 is also accompanied by changes in morphology.

### Combination of P2Y and P2X receptor antibodies

P2X are fast acting ligand (ATP)-gated ion channels that open the cell to ions, particularly calcium. P2Y receptors are much slower acting metabotropic receptors that act on internal cellular calcium stores following activation by ATP or UTP or even ADP by triggering a G-protein coupled cascade of activity and so function entirely differently to P2X receptors.

When P2X receptor antibodies were used in combination with P2Y receptor antibodies, a similar pattern to that described using P2Y receptor antibodies alone was obtained. In particular, labelling of prostate cells/tissues using antibodies against P2Y₂ and either or both P2X₁ and P2X₂ receptor subtypes enhanced results obtained using antibodies against the P2Y₂ receptor alone.

P2X antibodies were produced as follows:

The consensus sequences of the human P2X₁[17], human P2X₂[18], human P2X₃ [19], human P2X₄[20], human P2X₅[21], human P2X₆[22] and human P2X₇[23] were examined for suitable epitopes following the approach used for the rat antibodies [15, 24]. The non-homologous epitopes corresponding to the segment Lys68-Val84 used in P2X₁ with an added N-terminal Cys. His209-Cys226 was selected from P2X₂. Asn185-Cys203 was selected in P2X₃. Cys270-Glu285 was selected from P2X₄. Cys272-Ser288 was selected from P2X₅. Asn200-Cys218 was selected from P2X₆. Va165-Lys81 to which was added a C-terminal Cys was selected from P2X₇. All epitopes were conjugated to diptheria toxin via maleimidocaproyl-N-hydroxysuccinimide for crosslinking to diphtheria toxin. Antibodies were raised in rabbits and sheep and purified as described above for the P2Y antibodies.

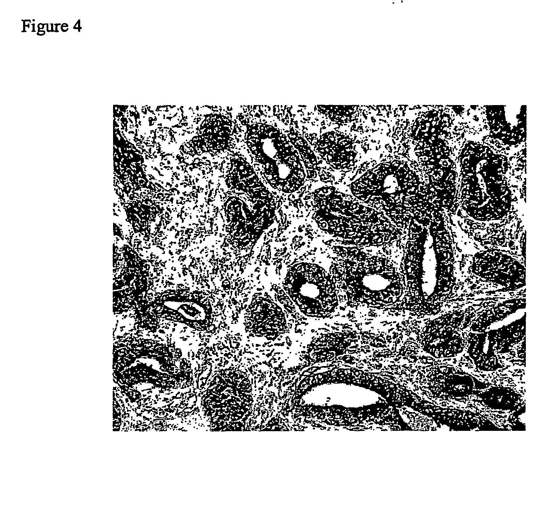

Figure 4 shows the use of a combination of P2Y₂, P2X₁ and P2X₂ antibody labelling of prostate tissue from a stage 3 case (obvious cancer).

The use of other P2X subtypes such as P2X₃ and P2X₇ yielded similar results in prostate samples.

### Example 4 - P2Y receptors in human cancer tissue (breast and skin)

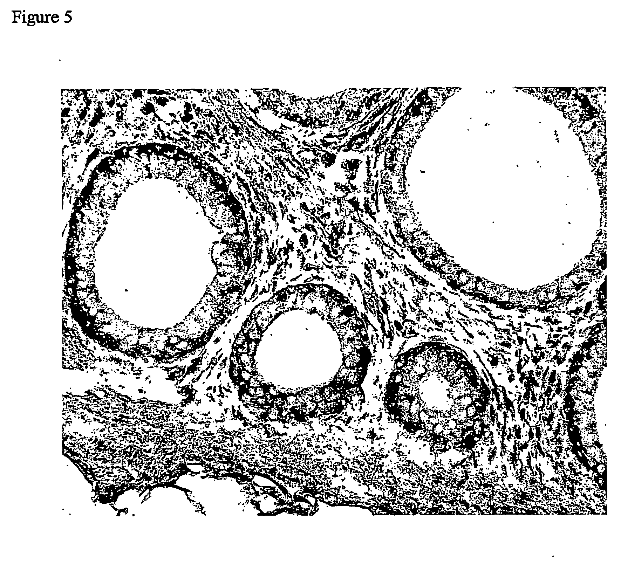

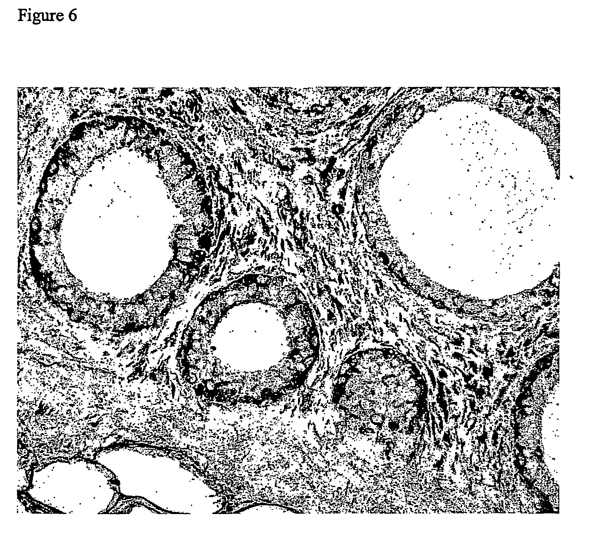

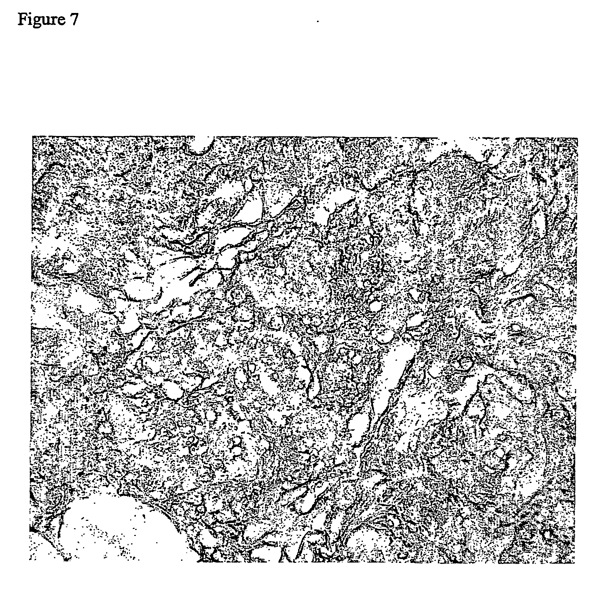

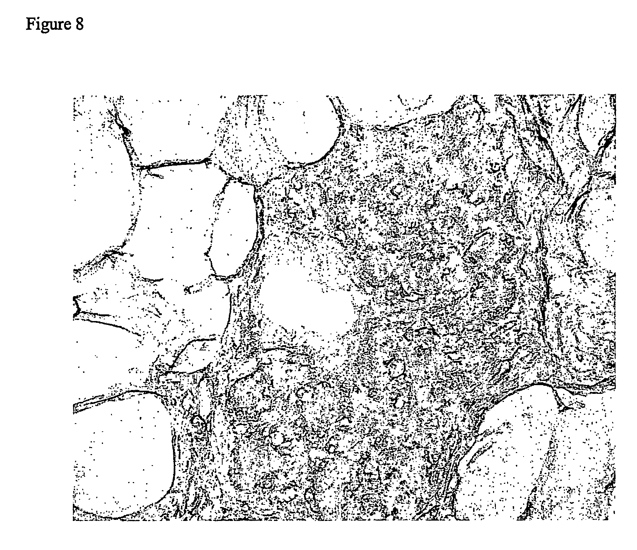

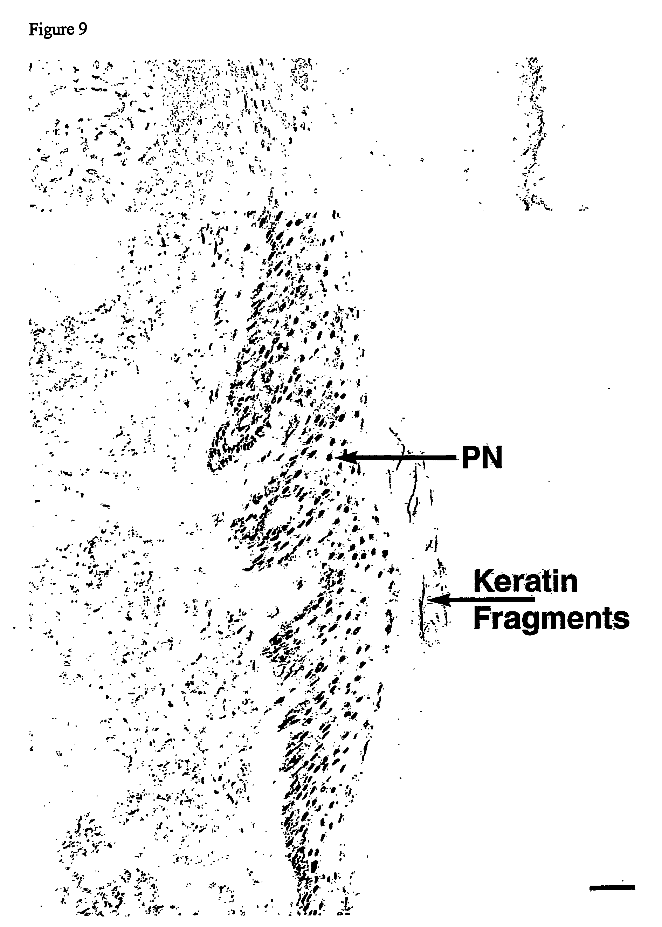

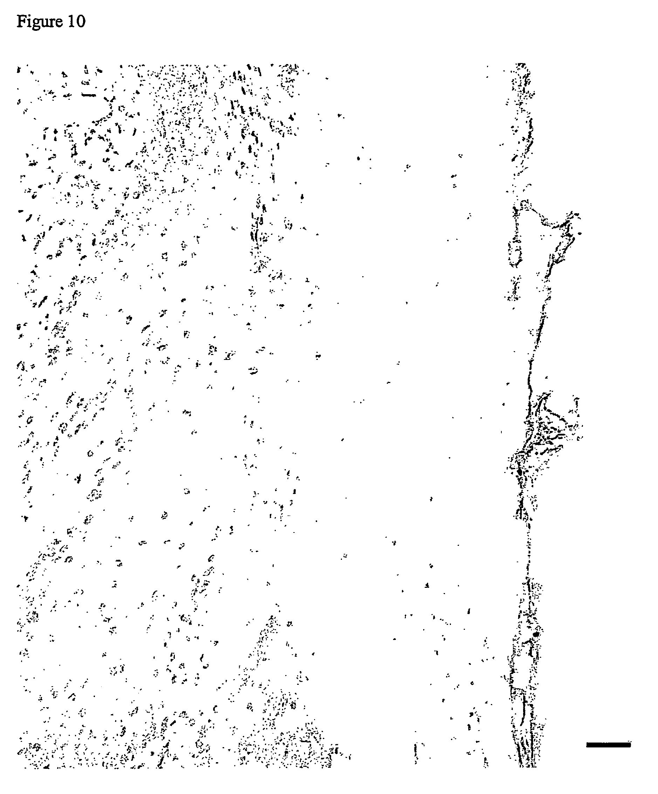

The labelling patterns were reversed in other cancers such as breast cancers (single and multi-foci) and skin cancers including malignant melanoma. In these tissues, P2Y₂ antibody labelling of normal tissue was intense (implying that the expression of P2Y₂ in normal tissue was relatively high) but P2Y₂ antibody labelling was not observed in cancer tissue. Results were enhanced by the use of antibodies against P2X receptors in combination with antibodies against P2Y₂ receptor, in particular antibodies against P2X₁ and P2X₂ subtypes (Figures 5 to 10).

### Example 5 - Combining P2Y receptor antibody diagnosis with other tests

It is clear that the P2Y (and P2X) receptor antibody diagnosis described above can be combined with complementary tests. Such tests may provide further information as to the nature of the cancer eg. a test for malignancy such as the anti-telomerase antibody test, and/or anti-tenascin may be useful in devising treatment regimes.

Although the invention has been described with reference to specific examples, it will be appreciated by those skilled in the art that the invention may be embodied in many other forms.

### References

**1.** Lian FR, Bhuiyan M, Li YW, Wall N, Kraut M, and Sarkar FH, 1998 Genistein-Induced G(2)-M Arrest, P21(Wafl) Upregulation, and Apoptosis in a Non-Small-Cell Lung Cancer Cell Line. Nutr. & Cancer. 31:184-191.
**2.** Hoey J, 1998 Prostate cancer: progress and perplexity. CMAJ 159:1-3.
**3.** Festuccia C, Vincentini C, di Pasquale AB, Aceto G, Zazzeroni F, Miano L, and Bologna M, 1995 Plasminogen activator activities in short-term tissue cultures of benign prostatic hyperplasia and prostatic carcinoma. Oncol. Res. 7:131-138.
**4.** Saxena S, Mohanty NK, and Jain AK, 1997 Screening of prostate cancer in males with prostatism. Ind. J. Pathol. Micro. 40:441-450.
**5.** Diamandis EP and Yu H, 1997 Nonprostatic sources of prostate-specific antigen. Urol. Clin. Nth. Am. 24:275-282.
**6.** Weyler J, 1999 Prostate cancer: screening or watchful waiting? Ann. Oncol. 9:9-11.
**7.** Bassler TJ, Orozco R, Bassler IC, Odowd GJ, and Stamey TA, 1998 Most Prostate Cancers Missed By Raising the Upper Limit of Normal Prostate-Specific Antigen For Men in Their Sixties Are Clinically Significant. Urol. 52:1064-1069.
**8.** Rabbani F, Stroumbakis N, Kava BR, Cookson MS, and Fair WR, 1998 Incidence and clinical significance of false-negative sextant prostate biopsies. J. Urol. 159:1247-1250.
**9.** Gao X, Porter AT, Grignon DJ, Pontes JE, and Honn KV, 1997 Diagnostic and prognostic markers for human prostate cancer. Prostate 31:264-281.
**10.** Small EJ, 1997 Prostate cancer. Curr. Opin. Oncol. 9:277-286.
**11.** Moul JW, Mooneyhan RM, Kao TC, McLeod DG, and Cruess DF, 1998 Preoperative and Operative Factors to Predict Incontinence, Impotence and Stricture After Radical Prostatectomy. Prost. Can. & Prost. Dis. 1:242-249.
**12.** Dasari VR, Sandhu AK, Mills DC, Athwal RS and Kunapuli SP, 1996 Mapping of the P2U purinergic receptor gene to human chromosome 11q 13.5-14.1 Somat. Cell Mol. Genet. 22, 75-79.
**13.** Communi D, Pirotton S, Parmentier M and Boeynaems JM, 1995 Cloning and functional expression of a human uridine nucleotide receptor J. Biol. Chem. 270, 30849-30852.
**14.** Maier R, Glatz A, Mosbacher J and Bilbe G, 1997 Cloning of P2Y6 cDNAs and identification of a pseudogene: comparison of P2Y receptor subtype expression in bone and brain tissues Biochem. Biophys. Res. Commun. 237, 297-302.
**15.** Hansen MA, Barden JA, Balcar VJ, Keay KA, and Bennett MR, 1997 Structural motif and characteristics of the extracellular domain of P2X receptors. Biochem. Biophys. Res. Comm. 236:670-675.
**16.** Barden JA, Cuthbertson RM, Jia-Zhen W, Moseley JM, and Kemp BE, 1997 Solution structure of parathyroid hormone related protein (residues 1-34) containing an Ala substituted for an Ile in position 15 (PTHrP[Ala15]-(1-34)). J. Biol. Chem. 272:29572-29578.
**17.** Valera S, Talabot F, Evans RJ, Gos A, Antonarakis SE, Morris MA and Buell GN, 1995 Characterization and chromosomal localization of a human P2X receptor from the urinary bladder. Recept. Channels 3, 283-289.
**18.** Lynch KJ, Touma E, Niforatos W, Kage KL, Burgard EC, van Biesen T, Kowaluk EA and Jarvis MF, 1999 Molecular and Functional Characterization of Human P2X(2) Receptors. Mol. Pharmacol. 56, 1171-1181.
**19.** Garcia-Guzman M, Stuhmer W and Soto F,1997 Molecular characterization and pharmacological properties of the human P2X3 purinoceptor Brain Res. Mol. Brain Res. 47, 59-66.
**20.** Garcia-Guzman M, Soto F, Gomez-Hernandez JM, Lund PE and Stuhmer W, 1997 Characterization of recombinant human P2X4 receptor reveals pharmacological differences to the rat homologue. Mol. Pharmacol. 51, 109-118.
**21.** Le KT, Paquet M, Nouel D, Babinski K and Seguela P, 1997 Primary structure and expression of a naturally truncated human P2X ATP receptor subunit from brain and immune system. FEBS Lett. 418, 195-199.
**22.** Urano T, Nishimori H, Han H, Furuhata T, Kimura Y, Nakamura Y and Tokino T, 1997 Cloning of P2XM, a novel human P2X receptor gene regulated by p53 Cancer Res. 57,3281-3287.
**23.** Buell GN, Talabot F, Gos A, Lorenz J, Lai E, Morris MA and Antonarakis SE, 1998 Gene structure and chromosomal localization of the human P2X7 receptor Recept. Channels 5, 347-354.
**24.** Dutton J, Hansen M, Balcar V, Barden J, and Bennett MR, 1999 Development of P2X receptor clusters on smooth muscle cells in relation to nerve varicosities in the rat urinary bladder. J. Neurocytol. 28, 3-15.

## Claims

1. An in vitro method of diagnosing or staging a preneoplastic and/or neoplastic state in a mammal, or determining the aetiology of carcinogenesis in a mammal, comprising detection of the P2Y purinergic receptor expression profile of a cell and/or tissue isolated from said mammal and comparison of the profile with a predetermined expression profile of a normal cell and/or tissue.

2. A method according to claim 1 wherein the mammal is a human, horse, bovine animal, sheep, dog, cat, rat or mouse, preferably a human.

3. A method according to claim 1 or 2 wherein the cell and/or tissue is from a prostate, breast, skin or other epithelial source.

4. A method according to any one of claims 1 to 3 wherein the pre-neoplastic or neoplastic state is prostate pre-cancer or prostate cancer and an increase in the intensity of the P2Y₂ purinergic receptor expression profile in a prostate cell or prostate tissue, compared to the expression profile of a normal prostate cell or tissue, as herein defined, is diagnostic of the presence of prostate pre-cancer or prostate cancer.

5. A method according to any one of claims 1 to 3 wherein the pre-neoplastic or neoplastic state is breast pre-cancer or breast cancer and a decrease in the intensity of the P2Y₂ purinergic receptor expression profile in a breast cell and/or breast tissue obtained from the mammal compared to the expression profile of a breast cell and/or breast tissue from a normal breast, is diagnostic of the presence of breast pre-cancer or breast cancer.

6. A method according to any one of claims 1 to 3 wherein the pre-neoplastic or neoplastic state is skin pre-cancer or skin cancer and a decrease in the intensity of the P2Y₂ purinergic receptor expression profile in a skin cell and/or tissue from the mammal compared to the expression profile of a skin cell and/or tissue from normal skin, is diagnostic of the presence of skin pre-cancer or skin cancer.

7. A method according to claim 6 wherein the skin cancer is malignant melanoma, squamous cell carcinoma or basal cell carcinoma.

8. A method according to any one of claims 1 to 3 wherein the P2Y receptor is a P2Y₂, P2Y₄ and/or P2Y₆ receptor, preferably a P2Y₂ receptor.

9. A method according to any one of claims 1 to 8 wherein the pre-neoplastic state is an early pre-neoplastic state in which the P2Y receptor expression profile is altered in the nucleus of the cell.

10. A method according to any one of claims 1 to 8 wherein the pre-neoplastic state is an advanced pre-neoplastic state in which the P2Y receptor expression profile is a profile in which the P2Y receptors are in the cytoplasm and/or lateral membranes and/or basal membranes.

11. A method according to any one of claims 1 to 8 wherein the neoplastic state is a state in which the P2Y receptor expression profile is a profile wherein the P2Y receptors are on the apical membrane of an epithelial cell.

12. A method according to any one of claims 1 to 11 wherein the P2Y receptor expression profile is combined with the expression profile of other markers, preferably the expression profile of P2X₁ and/or P2X₂.

13. A method according to any one of claims 1 to 12 wherein the P2Y receptor expression profile is detected by immunohistochemical means, or Western or Northern blotting techniques, preferably by ELISA or RIA.

14. A method according to any one of claims 1 to 12 wherein the P2Y receptor expression profile is detected by a colorimetric assay.

15. Use of A P2Y purinergic receptor-specific antibody, for in vitro differentiating between a pre-neoplastic or neoplastic cell and/or tissue and a normal cell and/or tissue.

16. Use according to claim 15 wherein the antibody is specific for P2Y₂, P2Y₄ and/or P2Y₆ receptors, preferably wherein the antibody is specific for P2Y₂ receptor, and optionally wherein the antibody is a monoclonal antibody.

17. Use according to claim 15 or 16 wherein the antibody is combined with at least one other antibody, preferably a P2Y receptor-specific antibody or a P2X receptor-specific antibody, more preferably a P2X₁ or P2X₂ receptor-specific antibody, and optionally wherein the at least one other antibody is a monoclonal antibody.

18. Use of A suite of P2Y purinergic receptor-specific antibodies, for in vitro differentiating between a pre-neoplastic or neoplastic cell and/or tissue and a normal cell and/or tissue, comprising antibodies according to any one of claims 15 to 17.

19. A method according to any one of claims 1 to 14 combined with a complimentary test, preferably a test for malignancy, and more preferably wherein the test for malignancy is an antelomerase antibody test using a specific antibody against human telomerase protein I (hTP₁) or an anti-tenascin test.

20. A method according to any one of claims 1 to 14 wherein the cell and/or tissue from said mammal is proximal to a pre-neoplastic or neoplastic cell and is not itself pre-neoplastic or neoplastic.

## Patentansprüche

1. Ein in-vitro Verfahren zur Diagnostizierung oder Einstufung eines präneoplastischen und/oder neoplastischen Stadiums in einem Säugetier, oder Bestimmung der Krankheitsursache von Karzinogenese in einem Säugetier, umfassend die Detektierung des P2Y Purinergen-Rezeptor-Expressionsprofils einer Zelle und/oder eines Gewebes isoliert aus besagtem Säugetier und dem Vergleich des Profils mit einem vorbestimmten Expressionsprofil einer normalen Zellen und/oder eines normalen Gewebes.

2. Ein Verfahren nach Anspruch 1, **worin** das Säugetier ein Mensch, ein Pferd, ein Horntier, ein Schaf, ein Hund, eine Katze, eine Ratte oder eine Maus, vorzugsweise ein Mensch, ist.

3. Ein Verfahren nach Anspruch 1 oder 2, **worin** die Zelle und/oder das Gewebe von einer Prostata, einer Brust, einer Haut oder einer anderen Epithelquelle ist.

4. Ein Verfahren nach irgendeinem der Ansprüche 1 bis 3, **worin** das präneoplastische oder neoplastische Stadium ein Prostatapräkrebs oder Prostatkrebs ist und ein Anstieg in der Intensität des P2Y₂ Purinergen-Rezeptor-Expressionsprofils in einer Prostatazelle oder in einem Prostatagewebe verglichen zu dem Expressionsprofil einer normalen Prostatazelle oder eines normalen Prostatgewebes , wie hierin definiert, auf die Gegenwart eines Prostatapräkrebses oder Prostatakrebses hindeutet.

5. Ein Verfahren nach irgendeinem der Ansprüche 1 bis 3, **worin** das präneoplastische oder neoplastische Stadium Brustpräkrebs oder Brustkrebs ist und ein Anstieg in der Intensität des P2Y₂ Purinergen-Rezeptor-Expressionsprofils in einer Brustzelle und/oder in einem Brustgewebe erhalten von dem Säugetier verglichen mit dem Expressionsprofil einer Brustzelle und/oder eines Brustgewebes einer normalen Brust auf die Gegenwart eines Brustpräkrebses oder Brustkrebses hindeutet.

6. Ein Verfahren nach irgendeinem der Ansprüche 1 bis 3, **worin** das präneoplastische oder neoplastische Stadium ein Hautpräkrebs oder ein Hautkrebs ist und ein Anstieg in der Intensität des P2Y₂ Purinergen-Rezeptor-Expressionsprofils in einer Hautzelle und/oder eines Hautgewebes eines Säugetiers verglichen mit dem Expressionsprofil einer Hautzelle und/oder eines Hautgewebes einer normalen Haut auf die Gegenwart eines Hautpräkrebses oder Hautkrebses hindeutet.

7. Ein Verfahren nach Anspruch 6, **worin** der Hautkrebs ein bösartiges Melanom, Plattenepithelkarzinom oder Basalzellenkarzinom ist.

8. Ein Verfahren nach irgendeinem der Ansprüche 1 bis 3, **worin** der P2Y-Rezeptor ein P2Y₂, P2Y₄ und/oder P2Y₆-Rezeptor, vorzugsweise ein P2Y₂-Rezeptor, ist.

9. Ein Verfahren nach irgendeinem der Ansprüche 1 bis 8, **worin** das präneoplastische Stadium ein frühes präneoplastisches Stadium ist, in welchem sich das P2Y-Rezeptor-Expressionsprofil im Nucleous der Zelle ändert.

10. Ein Verfahren nach irgendeinem der Ansprüche 1 bis 8, **worin** das präneoplastische Stadium ein fortgeschrittenes präneoplastisches Stadium ist, in welchem das P2Y-Rezeptor-Expressionsprofil ein Profil ist, in welchem die P2Y-Rezeptoren in dem Zytoplasma und/oder den Lateralmembranen und/oder den Basalmembranen sind.

11. Ein Verfahren nach irgendeinem der Ansprüche 1 bis 8, **worin** das neoplastische Stadium ein Stadium ist, in welchem das P2Y-Rezeptor-Expressionsprofil ein Profil ist, in welchem die P2Y-Rezeptoren auf der Apikalmembran einer Epithelzelle sind.

12. Ein Verfahren nach irgendeinem der Ansprüche 1 bis 11, **worin** das P2Y-Rezeptor-Expressionsprofil mit dem Expressionsprofil anderer Marker, vorzugsweise dem Expressionsprofil von P2X₁ und/oder P2X₂, kombiniert ist.

13. Ein Verfahren nach irgendeinem der Ansprüche 1 bis 12, **worin** das P2Y-Rezeptor-Expressionsprofil mittels immunhistochemischer Mittel oder Western- oder Northem Blott-Techniken, vorzugsweise mittels ELISA oder RIA, detektiert wird.

14. Ein Verfahren nach irgendeinem der Ansprüche 1 bis 12, **worin** das P2Y-Rezeptor-Expressionsprofil durch ein kalometrisches Essay detektiert wird.

15. Verwendung eines P2Y-Purinergen-Rezeptor spezifischen Antikörpers für in-vitro Differenzierung zwischen einer präneoplastischen oder neoplastischen Zelle und/oder Gewebes und einer normalen Zelle und/oder eines normalen Gewebes.

16. Verwendung nach Anspruch 15, **worin** der Antikörper spezifisch für P2Y₂, P2Y₄ und/oder P2Y₆-Rezeptoren ist, vorzugsweise worin der Antikörper spezifisch für P2Y₂-Rezeptoren ist, und optional worin der Antikörper ein monoklonaler Antikörper ist.

17. Verwendung nach Anspruch 15 oder 16, **worin** der Antikörper mit mindestens einem anderen Antikörper, vorzugsweise einem P2Y-Rezeptor spezifischen Antikörper oder einem P2X-Rezeptor spezifischen Antikörper noch mehr bevorzugt mit einem P2X₁ oder P2X₂-Rezeptor spezifischen Antikörper kombiniert ist und optional worin zumindest ein anderer Antikörper ein monoklonaler Antikörper ist.

18. Verwendung einer Reihe von Purinergen-Rezeptor spezifischen Antikörpern für in-vitro Differenzierung zwischen einer präneoplastischen oder neoplastischen Zelle und/oder Gewebes und einer normalen Zelle und/oder eines normalen Gewebes umfassend Antikörper nach irgendeinem der Ansprüche 15 bis 17.

19. Ein Verfahren nach irgendeinem der Ansprüche 1 bis 14 kombiniert mit einem Komplimentärstest, vorzugsweise ein Test auf Malignität, und besonders vorzugsweise worin der Test für Malignität ein Antelomerase-Antikörpertest unter Verwendung eines spezifischen Antikörpers gegen menschliches Telomeraseprotein I (hTP₁) oder ein Antitenasin-Test ist.

20. Ein Verfahren nach irgendeinem der Ansprüche 1 bis 14, **worin** die Zelle und/oder das Gewebe des besagten Säugetiers proximal zu der präneoplastischen oder neoplastischen Zelle ist und selbst nicht präneoplastisch oder neoplastisch ist.

## Revendications

1. Procédé in vitro pour diagnostiquer ou activer un état pré-néoplasique et / ou néoplasique dans un mammifère ou pour déterminer l'étiologie de la carcinogénèse dans un mammifère, comprenant la détection du profil d'expression des récepteurs purinergiques P2Y d'une cellule et / ou d'un tissu isolé dudit mammifère et la comparaison du profil avec un profil d'expression prédéterminé d'une cellule normale et / ou d'un tissu normal.

2. Procédé selon la revendication 1, dans lequel le mammifère est un être humain, un cheval, un bovin, un mouton, un chien, un chat, un rat ou une souris, de préférence un être humain.

3. Procédé selon la revendication 1 ou 2, dans lequel la cellule et / ou le tissu provient d'une prostate, d'un sein, d'une peau ou d'une autre source épithéliale.

4. Procédé selon l'une quelconque des revendications 1 à 3, dans lequel l'état pré-néoplasique ou néoplasique est un pré-cancer de la prostate ou un cancer de la prostate et une augmentation de l'intensité du profil d'expression des récepteurs purinergiques P2Y₂ dans une cellule prostatique ou un tissu prostatique, comparé au profil d'expression d'une cellule prostatique normale ou d'un tissu prostatique normal, tel que défini ici, est le diagnostic de la présence d'un pré-cancer ou d'un cancer de la prostate.

5. Procédé selon l'une quelconque des revendications 1 à 3, dans lequel l'état pré-néoplasique ou néoplasique est un pré-cancer du sein ou un cancer du sein et une diminution de l'intensité du profil d'expression des récepteurs purinergiques P2Y₂ dans une cellule mammaire et / ou un tissu mammaire provenant du mammifère, comparé au profil d'expression d'une cellule mammaire et / ou d'un tissu mammaire provenant d'un sein normal, est le diagnostic de la présence d'un pré-cancer ou d'un cancer du sein.

6. Procédé selon l'une quelconque des revendications 1 à 3, dans lequel l'état pré-néoplasique ou néoplasique est un pré-cancer de la peau ou un cancer de la peau et une diminution de l'intensité du profil d'expression des récepteurs purinergiques P2Y₂ dans une cellule et / ou un tissu de la peau provenant du mammifère, comparé au profil d'expression d'une cellule de la peau et / ou d'un tissu provenant d'une peau normale, est le diagnostic de la présence d'un pré-cancer ou d'un cancer de la peau.

7. Procédé selon la revendication 6, dans lequel le cancer de la peau est un mélanome malin, un carcinome à cellule squameuse ou un carcinome baso-cellulaire.

8. Procédé selon l'une quelconque des revendications 1 à 3, dans lequel le récepteur P2Y est un récepteur P2Y₂, P2Y₄ et / ou P2Y₆, de préférence un récepteur P2Y₂.

9. Procédé selon l'une quelconque des revendications 1 à 8, dans lequel l'état pré-néoplasique est un état pré-néoplasique précoce dans lequel le profil d'expression des récepteurs P2Y est modifié dans le noyau de la cellule.

10. Procédé selon l'une quelconque des revendications 1 à 8, dans lequel l'état pré-néoplasique est un état pré-néoplasique avancé dans lequel le profil d'expression des récepteurs P2Y est un profil dans lequel les récepteurs P2Y sont dans le cytoplasme et / ou les membranes latérales et / ou les membranes basales.

11. Procédé selon l'une quelconque des revendications 1 à 8, dans lequel l'état pré-néoplasique est un état pré-néoplasique dans lequel le profil d'expression des récepteurs P2Y est un profil dans lequel les récepteurs P2Y sont sur la membrane apicale d'une cellule épithéliale.

12. Procédé selon l'une quelconque des revendications 1 à 11, dans lequel le profil d'expression des récepteurs P2Y est combiné avec le profil d'expression d'autres marqueurs, de préférence le profil d'expression de P2X₁ et / ou de P2X₂.

13. Procédé selon l'une quelconque des revendications 1 à 12, dans lequel le profil d'expression des récepteurs P2Y est détecté par un moyen immuno-histochimique ou par des techniques de buvardage Western ou Northern, de préférence ELISA ou RIA.

14. Procédé selon l'une quelconque des revendications 1 à 12, dans lequel le profil d'expression des récepteurs P2Y est détecté par un dosage colorimétrique.

15. Utilisation d'un anticorps spécifique des récepteurs purinergiques P2Y pour la différentiation in vitro entre une cellule et / ou un tissu pré-néoplasique ou néoplasique et une cellule normale et / ou un tissu normal.

16. Utilisation selon la revendication 15, dans laquelle l'anticorps est spécifique des récepteurs P2Y₂, P2Y₄ et / ou P2Y₆, de préférence dans laquelle l'anticorps est spécifique des récepteurs P2Y₂ et, éventuellement, dans laquelle l'anticorps est un anticorps monoclonal.

17. Utilisation selon la revendication 15 ou 16, dans laquelle l'anticorps est combiné avec au moins un autre anticorps, de préférence un anticorps spécifique des récepteurs P2Y ou un anticorps spécifique des récepteurs P2X, de manière davantage préférée un anticorps spécifique des récepteurs P2X₁ ou P2X₂ et éventuellement, dans laquelle ledit au moins un autre anticorps est un anticorps monoclonal.

18. Utilisation d'une suite d'anticorps spécifiques des récepteurs purinergiques P2Y pour la différentiation in vitro entre une cellule et / ou un tissu pré-néoplasique ou néoplasique et une cellule normale et / ou un tissu normal, comprenant des anticorps selon l'une quelconque des revendications 15 à 17.

19. Procédé selon l'une quelconque des revendications 1 à 14 combiné avec un test complémentaire, de préférence un test de malignité et, de manière davantage préférée, dans lequel le test de malignité est un test à anticorps à activité anti-télomérase utilisant un anticorps spécifique contre la protéine télomérase humaine 1 (hTP₁) ou un test anti-ténascine.

20. Procédé selon l'une quelconque des revendications 1 à 14, dans lequel la cellule et / ou le tissu dudit mammifère est proximal(e) à une cellule pré-néoplasique ou néoplasique et n'est pas lui-même pré-néoplasique ou néoplasique.
